# EUROPÄISCHE PATENTSCHRIFT

(11) **EP 0 359 155 B1**
(45) Veröffentlichungstag und Bekanntmachung des Hinweises auf die Patenterteilung: **02.02.1994**
(21) Anmeldenummer: 89116703.3
(22) Anmeldetag: 09.09.1989
(51) Int. Cl.: C12Q 1/32, C12N 9/02

(54) **Verfahren zur Bestimmung von alpha-Ketoisocaproat in Körperflüssigkeitsproben und dafür geeigneter Analysensatz**
Method for the determination of alpha-ketoisocaproate in body fluid samples, and test kit suited therefor
Méthode pour la détermination d'alpha cétoisocaproate dans les échantillons de fluide corporel et trousse de réactifs pour la réalisation de cette méthode

(30) Priorität: 16.09.1988 DE 3831450
(43) Veröffentlichungstag der Anmeldung: 21.03.1990
(73) Patentinhaber: FORSCHUNGSZENTRUM JÜLICH GMBH, 52425 Jülich (DE)
(72) Erfinder: Hummel, Werner, Dr., D-5177 Titz (DE); Kula, Maria-Regina, Prof., D-5162 Niederzier-Hambach (DE); Wendel, Udo, Prof. Dr., D-4010 Hilden (DE); Schadewaldt, Peter, Dr., D-4000 Düsseldorf 13 (DE)

(56) Entgegenhaltungen:
- EP-A- 0 130 288
- CLIN. CHIM. ACTA, Band 183, Nr. 2, 15. August 1989, Elsevier Science Publishers B.V. (Biomedical Division); P. SCHADEWALDT et al., Seiten 171-182#
- ANALYTICAL BIOCHEMISTRY, Band 162, 1987, Academic Press Inc.; G.W. GOODWIN et al., Seiten 536-539#
- PEDIATRIC RESEARCH, Band 18, Nr. 9, 1984, Internatiol Pedriatic Research Foundation Inc., US; S.E. SNYDERMAN et al., Seiten 851-853#

## Beschreibung

Die Erfindung bezieht sich auf ein Verfahren zur selektiven Bestimmung des α-Ketoisocaproatgehalts von Körperflüssigkeiten, insbesondere Serumproben, durch enzymatische Reaktion in Gegenwart von Coenzym, und sie umfaßt einen dafür geeigneten Analysensatz.

Ketoisocaproat (KIC) ist ein Metabolit, welcher im Abbauweg von Leucin entsteht, normalerweise aber weiter abgebaut wird. Mitunter wird bei Neugeborenen ein als Ahornsirup-Krankheit (Leucinose) bezeichneter genetisch bedingter Defekt der verzweigtkettigen Ketosäure-Dehydrogenase beobachtet, bei dem der weitere Abbau von KIC sowie der Abbau der beiden anderen verzweigtkettigen Ketosäuren (gebildet aus Valin bzw. Isoleucin) blockiert ist. KIC und die anderen verzweigtkettigen Ketosäuren häufen sich im Serum und nachfolgend auch im Urin an. KIC hat eine toxische Wirkung auf das Gehirn und kann bei Vorliegen in hoher Konzentration zum Tod der Kinder im Säuglingsalter führen oder zu einer gestörten Gehirnentwicklung.

Die einzige erfolgreiche Therapie besteht in einer Ernährung mit einer Spezialdiät, in der die Zufuhr von Leucin und der anderen beiden verzweigtkettigen Aminosäuren Valin und Isoleucin in der Nahrung deutlich gegenüber der Norm eingeschränkt ist. Zur Überwachung der Therapie sind engmaschige Kontrollen der Plasmaleucin- oder -KIC-Spiegel erforderlich. Eine Besonderheit dieser Erkrankung ist, daß es bei fieberhaften Infekten, wie sie im Kindesalter sehr häufig auftreten, ebenfalls zu einem oftmals gefährlichen Anstieg von Leucin und KIC im Blut kommt. Essentielle Voraussetzung für eine wirkungsvolle Therapie ist eine gute Aminosäuren-Analytik oder KIC-Analytik. Zur patientennahen Stoffwechselüberwachung bietet sich insbesondere die KIC-Bestimmung im Urin an. Hierfür ist eine gute KIC-Analytik unbedingt Voraussetzung.

Bei anderen Stoffwechselanomalien und speziell bei Diabetes und Leberschädigungen kann es ebenfalls zu einer gefährlichen Akkumulation der physiologisch bedenklichen KIC kommen, so daß eine selektive Bestimmung derselben von erheblicher Bedeutung ist.

Das Problem der Analytik von KIC liegt darin, daß im Serum wie auch im Urin ebenfalls höhere Konzentrationen der beiden anderen verzweigtkettigen Ketosäuren (Ketomethylvalerat = KMV und Ketoisovalerat = KIV) vorkommen, die chemisch dem KIC sehr ähnlich sind und mit der KIC-Bestimmung interferieren.

Es gibt daher eine umfangreiche Literatur (28 Veröffentlichungen in den letzten zehn Jahren), die sich mit einer chromatographischen Trennung dieser Ketosäuren (nach entsprechender Derivatisierung) durch Gaschromatographie oder HPLC-Chromatographie befaßt (z. B. J. Chromat. 400 (1987) 91 - 99). Diese chromatographischen Methoden sind empfindlich und zuverlässig, benötigten aber einen hohen apparativen Aufwand und sind recht zeitraubend.

Eine KIC-Kontrolle ist daher nur in speziellen Analytiklabors möglich.

Zwar ist es bekannt, daß verzweigtkettige 2-Ketocarbonsäuren durch enzymatische Umsetzung verläßlich, rasch und mit geringem Aufwand analysiert werden können, jedoch wurde bislang lediglich die summarische Konzentration der drei genannten Ketosäuren ermittelt (G. Livesey und P. Lund in Biochem. J. 188 (1980) 705-713, insbesondere S. 711; "Methods in Enzymatic Analysis" (Bergmeyer, H.K., ed.) Bd. VIII S. 318-329, insbesondere S. 324, Verlag Chemie, Weinheim 1985; sowie Anal. Biochem. 162 (1987) 536 - 539).

Ziel der Erfindung ist es daher, ein Verfahren zur differenzierenden KIC-Bestimmung in biologischen Proben zu entwickeln, das ohne großen Aufwand durchführbar ist und ohne Vortrennung ausreichend genaue Werte selektiv für die KIC-Konzentration trotz gleichzeitiger Anwesenheit von KMV und KIV liefert.

Das zu diesem Zweck entwickelte erfindungsgemäße Verfahren ist dadurch gekennzeichnet, daß die Bestimmung unmittelbar in der ggf. von Eiweiß befreiten Probenflüssigkeit mittels KIC-spezifischer D-α-Hydroxyisocapronsäure-Dehydrogenase aus dieses Enzym enthaltenden Stämmen von Lactobacillus casei in Gegenwart von NADH durchgeführt wird.

Dabei wird die zeitliche NADH-Abnahme durch Absorptions-messungen bei 340 nm bestimmt. Alternativ könnte die NAD⁺-Bildung (durch Farbreaktion mittels eines NAD⁺-verbrauchenden Reagenz)überwacht werden.

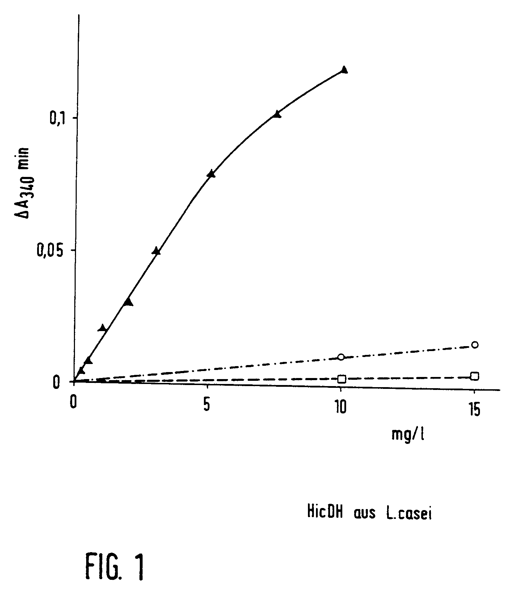

Die enzymatische Umsetzung von KIC mit der aus Lactobacillus casei isolierten KIC-spezifischen α-Hydroxy-isocapronsäure-Dehydrogenase verläuft überraschenderweise sehr viel schneller als bei KMV und KIV, wie durch die beigefügte Figur 1 veranschaulicht wird: Wie man sieht, können die enzymatischen Umsetzungen von KMV und KIV neben derjenigen von KIC in erster Näherung vernachlässigt werden.

Damit ist der Laboranalytik eine bequeme und rasche Methode zur Überwachung von KIC-Werten in organischen Proben, insbesondere Körperflüssigkeiten wie Blut oder Urin an die Hand gegeben.

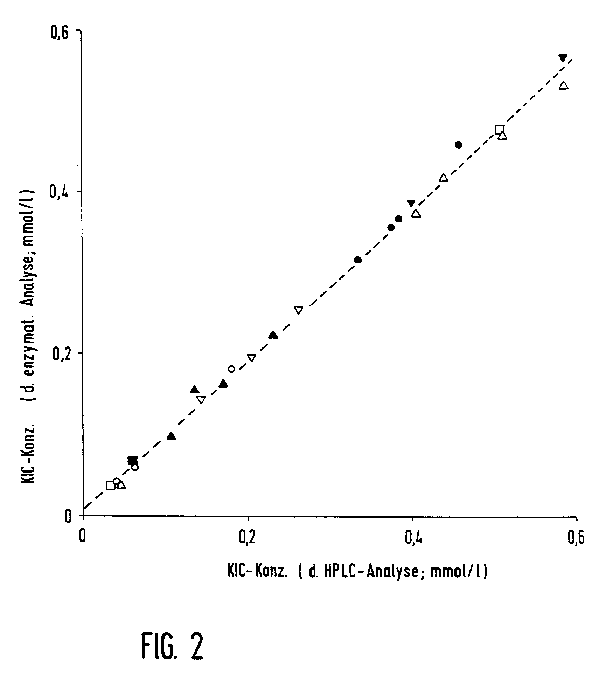

Eine Überprüfung der Analysenmethode durch parallele KIC-Bestimmung mit Hilfe der HPLC-Analyse ist in Figur 2 wiedergegeben: wie man sieht, liegen die Meßpunkte fast ideal auf der 45° Geraden, d. h. die sehr viel einfachere enzymatische Bestimmung steht der durch HPLC ermittelten Konzentrationsanalyse hinsichtlich der Aussagegenauigkeit nicht nach. Dabei wurden die Konzentrationsbestimmungen in praxisnaher Weise im Blut von Patienten mit Ahornsirupkrankheit ermittelt.

Die KIC-spezifische Dehydrogenase wird speziell aus Lactobacillus casei erhalten und zwar aus solchen Stämmen, die dieses Enzym bilden. Diese Eigenschaft kann durch einen relativ einfachen Test ermittelt werden:

Der kultivierte Stamm wird aufgeschlossen und der durch Zentrifugation erhaltene zellfreie Überstand für den Enzymtest verwendet.

Unter den z.Zt. verfügbaren Stämmen wurden auf diese Weise speziell Lactobacillus casei ssp. pseudoplantarum und ssp. casei als tauglich erkannt.

Vorzugsweise wird beim erfindungsgemäßen Verfahren D-α-Hydroxy-isocapronsäure-Dehydrogenase (DicDH) verwendet, die aus dem Lactobacillus casei ssp. pseudoplantarum und speziell aus dem Stamm (DSM 20008; Deutsche Sammlung von Mikroorganismen, Göttingen) isoliert wurde.

Die enzymatische Aktivität sollte insbesondere ≳ 5 U/mg betragen, wie sie insbesondere durch eine kombinierte Reinigung durch Phasenverteilung und Diafiltration erreicht wird, wodurch niedermolekulare Bestandteile entfernt werden. Auf diese Weise werden mindestens 90 % Fremdprotein abgetrennt und so störende Enzyme und Metabolite ausgeschaltet.

Ein Verfahren zur Gewinnung von D-α-Hydroxy-isocapronsäure-Dehydrogenase ist in der EP-A 0 130 288 beschrieben, in der die Gewinnung und Reinigung dieser Enzymgruppe aus unterschiedlichen Mikroorganismen näher angegeben ist. Gemäß vorliegender Erfindung wird jedoch nur aus Lactobacillus casei isoliertes Enzym angewandt und damit eine überraschend einfache KIC-Bestimmung in Körperflüssigkeit ohne besondere Vortrennung erreicht, bei der Störungen durch Begleitstoffe, insbesondere die zum KIC verwandten Ketosäuren nicht auftreten. Eine derart erhebliche Verbesserung im Bereich der Diagnose der Ahornsirup-Krankheit war aus der EP-PS offensichtlich nicht ohne weiteres herleitbar.

Nachfolgend wird die Erfindung anhand von Beispielen näher erläutert:

### Beispiel 1:

Die Auswahl des Enzyms basiert auf folgenden Untersuchungen und Ergebnissen:
Die Mikroorganismen-Art Lactobacillus casei wird zur Zeit in 5 Untergruppen (Subspecies, ssp.) unterteilt. Aus jeder Untergruppe wurde jeweils der Typstamm getestet, ob die entsprechende Hydroxysäure-Dehydrogenase geeignet ist, KIC selektiv nachzuweisen. Die drei Ketosäuren KIC, KMV und KIV wurden in einer Konzentration von 0,15 mM (Endkonzentration im Test) eingesetzt. Geeignet sind Enzyme, die mit dieser Ketosäurenkonzentration eine gute Reaktion mit KIC und einen schwache oder gar keine Reaktion mit KMV und KIV zeigen.

Für den Testansatz wurden die unter üblichen Bedingungen gezüchteten Mikroorganismen geerntet, die Zellen durch mechanische Einwirkung aufgeschlossen und der durch Zentrifugieren erhaltene Überstand als "Enzymlösung" für den Test verwendet.

Neben den verschiedenen Lactobacillus casei-Stämmen wurden noch weitere Lactobacillus-Stämme in die Untersuchung mit einbezogen, für die zwei typische Vertreter in der nachfolgenden Tabelle aufgeführt sind.

Der Tetansatz enthielt:
0,1 M Kaliumphosphatpuffer, pH 7,0;
0,2mM NADH;
0,020 ml Enzymlösung (0,1 - 0,2 mg Protein);
0,15 mM Ketosäure (entsprechend der Tabelle).

Ermittelt wurde die Anfangsreaktionsgeschwindigkeit über Extinktionsmessungen bei 340 nm in Abhängigkeit von der Zeit.

(Angegeben wird die Reaktionsgeschwindigkeit in Internationalen Einheiten (IU/min)
In der nachfolgenden Tabelle sind die erhaltenen Ergebnisse zusammengefaßt:

**Tab.**

| Eignung verschiedener Lactobacillus-Stämme für die selektive, enzymatische KIC-Bestimmung. | | | | |
|---|---|---|---|---|
| Organismus | DSM-Nr. | KIC | KMV | KIV |
| L. casei ssp. pseudoplantarum | 20 008 | 1,37 | 0,12 | 0 |
| L. casei ssp. pseudoplantarum | 20 207 | 2,00 | 0 | 0,14 |
| L. casei ssp. casei | 20 011 | 1,07 | 0 | 0,08 |
| L. casei ssp. alactosus | 20 020 | 0,01 | 0 | 0 |
| L. casei ssp. rhamnosus | 20 021 | 0 | 0 | 0 |
| L. casei ssp. tolerans | 20 258 | 0 | 0 | 0 |
| L. curvatus | 20 019 | 1,45 | 1,40 | 0,62 |
| L. confusus | 20 196 | 0,64 | 0,90 | 0,11 |

Wie die Tabelle zeigt, ist das Enzym aus Stämmen von Lactobacillus casei ssp. pseudoplantarum und Lactobacillus casei ssp. casei für die selektive KIC-Bestimmung geeignet. In der anderen untersuchten Untergruppen von Lactobacillus casei-Stämmen wird dieses Enzym ganz offensichtlich nicht gebildet. Weitere Lactobacillus-Stämme, die ebenfalls untersucht wurden, waren für diese Analytik nicht brauchbar.

### Beispiel 2:

Zur Demonstration der Selektivität der erfindungsgemäßen KIC-Bestimmung wurde eine vergleichende Konzentrationsbestimmung der drei Ketosäuren KIC, KMV und KIV mittels HicDH aus Lactobacillus casei ssp. pseudoplantarum (DSM 20008) durchgeführt. Dabei wurden Standardlösungen der drei Ketosäuren mit jeweils bekannter Konzentration (0,2 bis 15 µg/ml Testansatz) verwendet.

Der Testansatz enthielt:
0,1 M Kaliumphosphatpuffer pH 7,0;
0,2 mM NADH;
0,1 unit D-HicDH;
abgestufte Konzentrationen an Ketosäure.

Gemessen wurden die Anfangsreaktionsgeschwindigkeiten ΔE/min) bei 340 nm. Diese wurden gegen die Ausgangskonzentration der Ketosäure aufgetragen (Fig. 1): Wie die Figure zeigt, ist die Nachweisempfindlichkeit für KIC mehr als 10-fach besser als für KMV und KIV. Diese Differenz sollte für eine selektive KIC-Bestimmung in Serum ausreichen.

### Beispiel 3:

Messung von KIC-Konzentrationen im Blutplasma.

Der KIC-Gehalt im Plasma von Gesunden und von an Ahornsirup-Krankheit Erkrankten wurde enzymatisch (mit HicDH) bestimmt und verglichen mit KIC-Werten, die mit einer chromatographischen Methode ermittelt wurden. Dazu wurden Plasmaproben (0,15 bis 0,45 ml) enteiweißt (Zusatz eines gleichen Volumenanteils an HClO₄; 1,6 mol/l), zentrifugiert und durch Zusatz von KHCO₃ (6 mol/l) neutralisiert. Nach erneuter Zentrifugation wurden 0,1 ml des Überstandes für den jeweiligen Test eingesetzt.

### Enzymatischer Test:

Der enzymatische Testansatz enthielt in einem Endvolumen von 1,04 ml:
0,1 M Kaliumphosphatpuffer, pH 6,5;
0,2 mM NADH;
0,015 units D-HicDH (in 20 µl);
0,1 ml enteiweißtes Plasma.

Im Falle, daß die KIC-Konzentration ≦ 0,1 mmol/l lag, wurden 0,5 ml Plasma eingesetzt und die Enzymmenge verdoppelt.

Die Anfangsreaktionsgeschwindigkeit wurde bei 334 nm am Eppendorf-Spektralphotometer bestimmt (temperiert auf 25°C). Mit Hilfe einer Eichkurve, die mit bekannten KIC-Konzentrationen erstellt worden war, konnte der KIC-Wert für die jeweilige Plasmaprobe ermittelt werden. Diese Plasmawerte wurden verglichen mit den KIC-Werten, die durch Enteiweißung, Chinoxalinol-Derivatisierung, HPLC und Fluoreszenznachweis (338 nm Interferenz-Anregungsfilter und 425 nm Emissionsfilter) anhand von Eichwerten erhalten wurden.

Figur 2 zeigt die enzymatisch und die chromatographisch gemessenen KIC-Werte in verschiedenen Plasmaproben: Untersucht wurden 24 Plasmaproben, darunter befanden sich 8 Proben von Patienten mit Ahornsirup-Krankheit. Figur 2 zeigt die sehr gute Korrelation beider KIC-Bestimmungsmethoden. Störungen durch weitere Plasmabestandteile sind nicht zu beobachten. Damit bietet die HicDH aus Lactobacillus casei eine gute Möglichkeit zur Diagnose und Überwachung der Ahornsirup-Krankheit.

Wesentliche Vorteile der enzymatischen Methode sind die schnelle Durchführung der Messung (2-3 min pro Messung, mit der Möglichkeit, mehrere Proben parallel zu bestimmen), ein geringer apparativer Aufwand (einfaches Einstrahlphotometer) und die Möglichkeit zur Automatisierung der Meßmethode (Anwendung von NAD-abhängigen Enzymen im Analysenautomaten ist Stand der Technik).

Selbstverständlich ist die erfindungsgemäße selektive KIC-Bestimmung nicht auf Blut- oder auch Urinanalysen von Patienten mit Ahornsirup-Krankheit beschränkt, vielmehr kann sie überall da eingesetzt werden, wo insbesondere durch Stoffwechselanomalien eine erhöhte KIC-Konzentration im biologischen Material auftritt, wie z. B. bei Diabetes, Leberzirrhose, Niereninsuffizienz usw.

## Patentansprüche

1. Verfahren zur selektiven Bestimmung des α-Ketoisocaproatgehalts von Körperflüssigkeiten, insbesondere Serumproben, durch enzymatische Reaktion in Gegenwart von Coenzym,
**dadurch gekennzeichnet,**
daß die Bestimmung unmittelbar in der ggf. von Eiweiß befreiten Probenflüssigkeit mittels KIC-spezifischer D-α-Hydroxyisocapronsäure-Dehydrogenase aus dieses Enzym enthaltenden Stämmen von Lactobacillus casei in Gegenwart von NADH durchgeführt wird.

2. Verfahren nach Anspruch 1,
**dadurch gekennzeichnet,**
daß man aus Lactobacillus casei ssp. pseudoplantarum oder casei isoliertes Enzym verwendet.

3. Verfahren nach Anspruch 1,
**dadurch gekennzeichnet,**
daß man aus dem Lactobacillus casei ssp. pseudoplantarum (DSM 20008) isolierte D-α-Hydroxy-isocapronsäure-Dehydrogenase verwendet.

4. Verfahren nach einem der Ansprüche 1 bis 3,
**dadurch gekennzeichnet,**
daß man die α-Ketoisocaproat-Bestimmung im Serum oder Urin von Patienten zur Diagnose der Ahornsirup-Krankheit durchführt.

5. Analysensatz für differenzierende KIC-Tests,
**gekennzeichnet durch**
einen standardisierten insbesonderen aufteilbaren Vorrat an D-α-Hydroxy-isocapronsäure-Dehydrogenase aus Lactobacillus casei, insbesondere aus dem Lactobacillus casei DSM 20008 und gegebenenfalls entsprechende NADH- und Puffer-Vorratsmengen.

## Claims

1. A method for the selective determination of the α-keto-isocaproate content of body fluids, particularly serum samples, by enzymatic reaction in the presence of a coenzyme, characterised in that the determination is carried out directly on the sample liquid, which is optionally free from protein, by means of KIC-specific D-α-hydroxycaproic acid dehydrogenase from strains of lactobacillus casei containing this enzyme, in the presence of NADH.

2. A method according to claim 1, characterised in that an enzyme isolated from lactobacillus casei ssp. pseudoplantarum or casei is used.

3. A method according to claim 1, characterised in that D-α-hydroxyisocaproic acid dehydrogenase isolated from lactobacillus casei ssp. pseudoplantarum (DSM 20008) is used.

4. A method according to any one of claims 1 to 3, characterised in that the α-keto-isocaproate determination is performed on the serum or urine of patients for the diagnosis of maple syrup urine disease.

5. An analysis kit for performing differentiating KIC tests, characterised by a standardised supply, particularly an apportionable supply, of D-α-hydroxyisocaproic acid dehydrogenase from lactobacillus casei, particularly from lactobacillus casei DSM 20008, and optionally by corresponding amounts of NADH and buffer supplies.

## Revendications

1. Procédé pour la détermination sélective de la quantité d' α-céto-isocaproate contenu dans des liquides corporels, en particulier dans des échantillons de sérum, grâce à une réaction enzymatique en présence d'une coenzyme, caractérisé en ce qu'on effectue cette détermination directement dans le liquide d'échantillonnage éventuellement débarrassé de la protéine, au moyen d'acide D-α-hydroxy-isocaproïque-déshydrogénase spécifique de KIC (céto-isocaproate) provenant de souches de Lactobacillus casei contenant cette enzyme, en présence de NADH.

2. Procédé selon la revendication 1, caractérisé en ce qu'on utilise une enzyme isolée de Lactobacillus casei ssp. pseudoplantarum ou casei.

3. Procédé selon la revendication 1, caractérisé en ce qu'on utilise l'acide D-α-hydroxy-isocaproïque-déshydrogénase isolée du Lactobacillus casei ssp. pseudoplantarum (DSM 20008).

4. Procédé selon l'une des revendications 1 à 3, caractérisé en ce qu'on effectue la détermination de l'α-céto-isocaproate dans du sérum ou dans l'urine de patients pour diagnostiquer la leucinose.

5. Set analytique pour des essais de différenciation de KIC, caractérisé par une réserve normalisée et notamment dosable d'acide D-α-hydroxy-isocaproïque-déshydrogénase provenant de Lactobacillus casei, notamment du Lactobacillus casei DSM 20008, et éventuellement de quantités de réserve de NADH et de tampon.
